# EUROPÄISCHE PATENTSCHRIFT

(11) **EP 0 269 846 B1**
(45) Veröffentlichungstag und Bekanntmachung des Hinweises auf die Patenterteilung: **21.12.1994**
(21) Anmeldenummer: 87115680.8
(22) Anmeldetag: 26.10.1987
(51) Int. Cl.: A61N 1/08

(54) **Reizstromgerät**
Stimulator
Stimulateur

(30) Priorität: 07.11.1986 DE 3638002
(43) Veröffentlichungstag der Anmeldung: 08.06.1988
(73) Patentinhaber: SIEMENS AKTIENGESELLSCHAFT, 80333 München (DE)
(72) Erfinder: Helmreich, Klaus, D-8520 Erlangen (DE); Herzog, Ludwig, D-6948 Waldmichelbach (DE); Knapp, Volker, D-6948 Waldmichelbach (DE)

(56) Entgegenhaltungen:
- EP-A- 0 170 365
- DE-A- 2 843 724
- DE-A- 3 216 911
- GB-A- 2 139 436
- US-A- 4 023 046
- MODERN ELECTRIC CIRCUITS MANUAL, Seite 1159, J.Markus, 1980 McGraw-Hill
- ELEKTOR, Juli/August 1986, Seiten 113-114
- Enzyklopädie Naturwissenschaft und Technik, Verlag Moderne Industrie 1980, Seite 2315
- Enzyklopädie Naturwissenschaft und Technik, Verlag Moderne Industrie 1979, Seiten 168 und 876

## Beschreibung

Die Erfindung bezieht sich auf ein Reizstromgerät für Reizstromtherapie an einem Patienten, mit einer auf einer vorgebbaren Betriebsspannung arbeitenden Endstufe, der ausgangsseitig ein Fensterdiskriminator zugeordnet ist, welcher ein Abschaltsignal für einen Abschalter zur Abschaltung eines inneren Stromkreises des Reizstromgerätes erzeugt, wenn der Gleichanteil der Spannung am Ausgang der Endstufe einen vorgebbaren Wert überschreitet.

Ein aus der DE-A-32 16 911 bekanntes Reizstromgerät dieser Art umfaßt einen Überwachungsblockstromkreis und einen Operationsverstärker, dessen beiden Eingänge über Gleichrichter und Widerstände gleichgerichtete und gewichtete Elektroden-Ausgangsspannungen und -Ströme zugeführt werden. Fließt bei Anliegen der Spannung an den Elektroden zu wenig Strom, dann wird das Gerät abgeschaltet. Dazu weist das Gerät zwei die Reizstromelektroden über ein Relais abschaltende Kontakte auf. Bei der bekannten Anordnung wird nur eine Spannungsgrenze überwacht.

Aus der Zeitschrift ELEKTOR, Juli/August 1986, Seiten 113-114, ist eine Netzspannungsanzeige zur Überwachung eines Spannungsabfalls, ausgelöst durch das Einschalten großer Verbraucher, wie Kühlschrank, während eines Computerbetriebes, bekannt. Aus einer stabilisierten Spannung von 15 V werden dabei zwei Referenzspannungen abgeleitet, die mit einer Meßspannung (Teil der Netzspannung) verglichen werden. Ist die Netzspannung zu gering, leuchtet eine erste LED auf, ist die Netzspannung zu hoch, zeigt dies eine zweite LED an.

Aus der EP-A-0 170 365 ist ein Reizstromgerät mit zwei auf einer vorgebbaren positiven Betriebsspannung arbeitenden Endstufen bekannt, welchen ausgangsseitig über je eine Diode der invertierende Eingang eines Komparators zugeordnet ist. Der Komparator erzeugt ein Abschaltsignal, das über einen Mikroprozessor je einem Eingang an je einer vor jeder der beiden Endstufen angeordneten Torschaltung zugeführt wird, wenn an einer der beiden Endstufen die positive Ausgangsspannung einen vorgebbaren prozentualen Wert der Betriebsspannung überschreitet. Dadurch wird je ein innerer Stromkreis an jeder der beiden Endstufen des bekannten Reizstromgerätes abgeschaltet.

Aus der GB-A-2 139 436 ist eine Schutzschaltung für Netzwechselstrom bekannt, die erkennt, ob die Netzspannung innerhalb eines Fensters ist oder nicht. Dazu enthält eine Vergleichsschaltung zwei Operationsverstärker. An einem invertierenden Eingang des einen Operationsverstärkers und an einem nicht invertierenden Eingang des anderen Operationsverstärkers ist über einen Eingangswiderstand eines ersten Spannungsteilers die gleichgerichtete positive Halbwelle der Sekundärwicklung eines Netztransformators zugeführt. Der nicht invertierende Eingang des einen Operationsverstärkers und der invertierende Eingang des anderen Operationsverstärkers sind über je einen weiteren Spannungsteiler (je zwei Festwiderstände, je ein Potentiometer) mit einer gleichgerichteten positiven weiteren Spannung der Sekundärwicklung des Netztransformators verbunden. An den beiden weiteren Spannungsteilern werden über je ein Potentiometer positive Spannungsschwellwerte eingestellt, die etwa ± 5 % bis ± 15 % des Spitzennennwertes der Netzspannung betragen, vorzugsweise 200 V bis 250 V bei einem Nennwert von 230 V. Wenn der eingestellte Fensterbereich von der Netzspannung verlassen wird, betätigt die Vergleichsschaltung über einen monostabilen Multivibrator ein Relais, das eine an die Schutzvorrichtung angeschlossene weitere Vorrichtung abschaltet.

Aus der Druckschrift MODERN ELECTRONIC CIRCUITS MANUAL; J.Markus, 1980 McGraw-Hill, Seite 1159, ist ein "Out-of-limit Voltage sensor" bekannt. Dieser Sensor umfaßt eine Hälfte des Quad Komparators HA-4900/4905. Der nicht invertierende Eingang des ersten Komparators und der invertierende Eingang des zweiten Komparators sind miteinander verbunden und tragen die Kennzeichnung "INPUT". Der invertierende Eingang des ersten Komparators trägt die Kennzeichnung "HI REF" und der nicht invertierende Eingang des zweiten Komparators trägt die Kennzeichnung "LO REF". Der Sensor kann zur Ansteuerung eines Alarmindikators benutzt werden. Die Ausgänge beider Komparatoren sind mit dem integrierten Schaltkreis HO74CO2 verbunden, der von einer Betriebsspannung V_{CC} gespeist wird. Der Ausgang dieser integrierten Schaltung HO-74202 ist mit "IN WINDOW" bezeichnet.

Bei einem Reizstromgerät der eingangs genannten Art liegt bei Konstantstrombetrieb, wenn das Gerät nicht abgeschaltet wird, an abgenommenen oder abgefallenen Elektroden die volle positive oder negative Betriebsspannung des Endverstärkers. Die Berührung der Elektroden ist wegen der momentanen, relativ großen Stromdichte an den Übergangsflächen zur Haut gewöhnlich schmerzhaft. Dasselbe kann im Konstantspannungsbetrieb auftreten, wenn z.B. die Endstufe defekt ist.

Aufgabe vorliegender Erfindung ist es, bei einem Reizstromgerät der eingangs genannten Art Mittel vorzusehen, die den inneren Stromkreis abschalten, wenn die Elektroden abgenommen werden oder abfallen.

Die Aufgabe wird erfindungsgemäß mit den Merkmalen gemäß Patentanspruch 1 gelöst.

Vorzugsweise soll das Gerät abschalten, wenn der Fensterdiskriminator einen Wert von etwa 90 % der Betriebsspannung registriert.

Weitere Vorteile und Einzelheiten der Erfindung ergeben sich aus der nachfolgenden Beschreibung eines Ausführungsbeispiels anhand der Zeichnung und in Verbindung mit den abhängigen Ansprüchen.

Es zeigen:
Figur 1 die Erfindung im Prinzipschaltbild,
Figur 2 ein Diagramm des Spannungsverlaufes am Ausgang der Endstufe in Abhängigkeit vom Widerstand zwischen den Elektroden und
Figur 3 ein Ausführungsbeispiel für den Fensterdiskriminator.

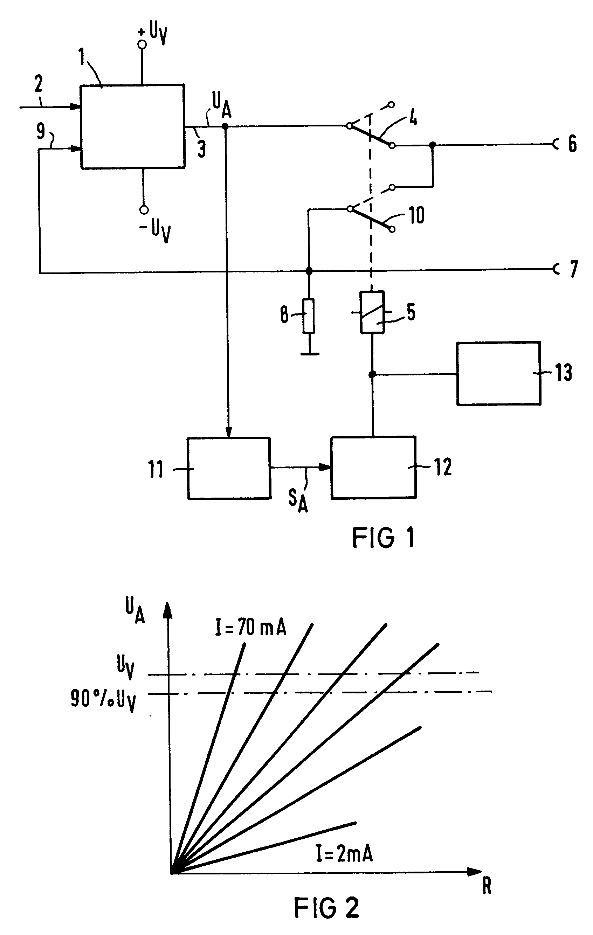

In der Figur 1 umfaßt das Reizstromgerät u.a. eine Endstufe 1 (Betriebsspannung ± U_{V} = 180 V) mit Signaleingang 2 und Signalausgang 3, der über einen ersten Schaltkontakt 4 eines Relais 5 mit der einen Elektrodenanschlußbuchse 6 des Gerätes verbunden ist. Die andere Elektrodenanschlußbuchse 7 ist über einen niederohmigen Strommeßwiderstand 8 (z.B. 20 Ohm) mit einem Regeleingang 9 der Endstufe 1 verbunden. Ein weiterer Schaltkontakt 10 verbindet kurzschlußmäßig die Elektrodenanschlußbuchsen 6 und 7, wenn das Relais 5 im Sinne einer Abschaltung des inneren Stromkreises des Gerätes aktiviert wird. In diesem Falle befinden sich beide Schaltkontakte 4, 10 des Relais 5 in der gestrichelten Schaltstellung.

Das Relais 5 wird gemäß der Figur 1 von einem Fensterdiskriminator 11 über eine bistabile Kippstufe 12 angesteuert. Der Fensterdiskriminator 11 ist eingangsseitig am Ausgang 3 der Endstufe 1 angeschaltet. Er ist so eingestellt, daß er ein Abschaltsignal S_{A} an seinem Ausgang erzeugt, wenn die Ausgangsspannung U_{A} des Endverstärkers 1 ca. 90 % der Betriebsspannung ± U_{V} überschreitet. Aufgrund dieses Abschaltsignales kippt die bistabile Kippstufe 12 und betätigt somit das Relais 5 im Sinne der Abschaltung des inneren Stromkreises über die Schaltkontakte 4, 10.

Bei Abnahme oder Abfallen der Elektroden wird also das Gerät immer abgeschaltet, sobald U_{A} ungefähr 90 % von ± U_{V} ist.

Mit dem Abschalten wird von der bistabilen Kippstufe 12 auch noch gleichzeitig ein optischer und akustischer Alarmgeber 13 angestoßen.

Die Figur 2 zeigt die Abhängigkeit der Ausgangsspannung U_{A} des Endverstärkers 1 vom Widerstand R zwischen den Elektroden bei vorgegebenem Stromwert I. Man sieht aus diesem Diagramm sofort, daß, wenn der Widerstand R sehr groß wird (z.B. Abfallen oder Abnahme der Elektroden), die Spannung U_{A} sehr rasch gegen die Betriebsspannung U_{V} geht.

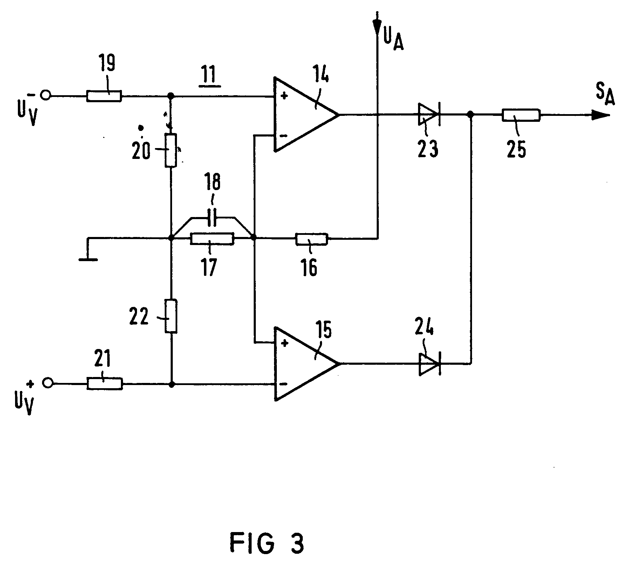

Die Figur 3 zeigt ein Ausführungsbeispiel für den Fensterdiskriminator 11. Er umfaßt für Gegentaktbetrieb einen ersten Operationsverstärker 14 und einen zweiten Operationsverstärker 15. Der invertierende Eingang des ersten und der nicht invertierende Eingang des zweiten Operationsverstärkers sind über einen ersten Spannungsteiler mit den Widerständen 16, 17 mit der Ausgangsspannung U_{A} der Endstufe verbunden. Das Bauelement 18 ist ein Kondensator, der momentane Spannungsspitzen, die nicht zu einer Abschaltung führen sollen, dämpft.

Der nicht invertierende Eingang des ersten Operationsverstärkers 14 ist über einen zweiten Spannungsteiler mit den ohmschen Widerständen 19, 20 mit der negativen Betriebsspannung -U_{V} verbunden. Der invertierende Eingang des zweiten Operationsverstärkers 15 liegt hingegen über einen dritten Spannungsteiler mit den ohmschen Widerständen 21, 22, die entsprechend den Widerständen 19, 20 dimensioniert sind, an der positiven Betriebsspannung +U_{V}.

Der Wert des Widerstandes 16 (z.B. 120 kOhm) beträgt etwa 90 % des Wertes der Widerstände 19 bzw. 21 (z.B. 133 kOhm). Die Widerstände 17, 20, 22 sind gleich groß (z.B. 5,1 kOhm). Dadurch überschreitet die Spannung am invertierenden Eingang des ersten Operationsverstärkers 14 die Spannung am nicht invertierenden Eingang, wenn |-U_{A}| größer 90 % von |-U_{V}| ist. Entsprechend überschreitet die Spannung am nicht invertierenden Eingang des zweiten Operationsverstärkers 15 die Spannung am invertierenden Eingang, wenn |+U_{A}| größer 90 % von |+U_{V}| ist. In beiden Fällen erzeugt der Fensterdiskriminator 11 über Dioden 23, 24 und einen ohmschen Widerstand 25 das Abschaltsignal S_{A}.

## Patentansprüche

1. Reizstromgerät für Reizstromtherapie an einem Patienten, mit einer auf einer vorgebbaren Betriebsspannung (U_{V}) arbeitenden Endstufe (1), der ausgangsseitig ein Fensterdiskriminator (11) zugeordnet ist, welcher ein Abschaltsignal (S_{A}) für einen Abschalter (4, 5, 10) zur Abschaltung eines inneren Stromkreises des Reizstromgerätes erzeugt, wenn der Gleichanteil der Spannung am Ausgang der Endstufe einen vorgebbaren Wert überschreitet, **dadurch gekennzeichnet,** daß eine auf einer vorgebbaren positiven und negativen Betriebsspannung (± U_{V}) im Gegentaktbetrieb arbeitende Endstufe (1) vorgesehen ist, wobei das Abschaltsignal (S_{A}) erzeugt wird, wenn eine positive oder eine negative Spannung (± U_{A}) am Ausgang der Endstufe (1) einen vorgebbaren prozentualen Betrag der positiven oder negativen Betiebsspannung (± U_{V}) überschreitet, wobei der Fensterdiskriminator (11) zwei Operationsverstärker (14, 15) umfaßt, deren Ausgänge zur Erzeugung des Abschaltsignals (S_{A}) über Dioden (23, 24) verbunden sind, wobei dem invertierenden Eingang des ersten Operationsverstärkers (14) und dem nicht invertierenden Eingang des zweiten Operationsverstärkers (15) die Ausgangsspannung (U_{A}) des Endverstärkers (1) über einen ersten Spannungsteiler (16, 17) und dem nicht invertierenden Eingang des ersten Operationsverstärkers (14) die negative Betriebsspannung (-U_{V}) über einen zweiten Spannungsteiler (19, 20) und dem invertierenden Eingang des zweiten Operationsverstärkers (15) die positive Betriebsspannung (+U_{V}) über einen dritten Spannungsteiler (21, 22), der entsprechend dem zweiten Spannungsteiler dimensioniert ist, zugeführt wird.

2. Reizstromgerät nach Anspruch 1, **dadurch gekennzeichnet,** daß der prozentuale Betrag ca. 90 % der Betriebsspannung (± U_{V}) ist.

3. Reizstromgerät nach Anspruch 1, **dadurch gekennzeichnet,** daß die Eingangswiderstände (19, 21) des zweiten und des dritten mit der negativen bzw. positiven Betriebsspannung (± U_{V}) verbundenen Spannungsteilers (19, 20; 21, 22) gleichdimensioniert sind und daß der Eingangswiderstand (16) des ersten mit der Ausgangsspannung der Endstufe (1) verbundenen Spannungsteilers (16, 17) zu jedem der Eingangswiderstände (19, 21) des zweiten und des dritten Spannungsteilers (19, 20; 21, 22) in einem Verhältnis steht, das dem vorgebbaren prozentualen Betrag der Betriebsspannung entspricht.

4. Reizstromgerät nach Anspruch 1, **dadurch gekennzeichnet,** daß der Fensterdiskriminator (11) den Abschalter (4, 5, 10) über eine bistabile Kippstufe (12) betätigt.

5. Reizstromgerät nach Anspruch 1, **dadurch gekennzeichnet,** daß das Abschaltsignal auch einen optischen und/oder akustischen Alarmgeber (13) aktiviert.

6. Reizstromgerät nach einem der Ansprüche 1 bis 5, **dadurch gekennzeichnet,** daß an der Endstufe (1) ein Signalausgang (3) über einen ersten Schaltkontakt (4) eines Relais (5) von einer Elektrodenanschlußbuchse (6) abschaltbar ist und ein weiterer Schaltkontakt (10) die Elektrodenanschlußbuchse (6) kurzschlußmäßig mit einer anderen Elektrodenanschlußbuchse (7) verbindet, wenn das Relais (5) im Sinne einer Abschaltung des inneren Stromkreises des Reizstromgerätes aktiviert wird.

## Claims

1. Stimulator for stimulus current therapy of a patient, having an end stage (1) operating at a pre-selected operating voltage (U_{V}), to which end stage there is assigned on the output side a window discriminator (11) which generates a tripping signal (S_{A}) for a cut-out (4, 5, 10) for disconnecting an inner electric circuit of the stimulator when the direct component of voltage exceeds a pre-selected value at the output of the end stage, characterised in that there is provided an end stage (1) which operates at a pre-selected positive and negative operating voltage (±U_{V}) in push-pull operation, wherein the tripping signal (S_{A}) is generated when a positive or negative voltage (±U_{A}) exceeds a pre-selected percentage amount of positive or negative operating voltage (±U_{V}), wherein the window discriminator (11) comprises two operational amplifiers (14, 15) whose outputs are connected by way of diodes (23, 24) for generating the tripping signal (S_{A}), wherein the output voltage (U_{A}) of the output amplifier (1) is supplied to the inverting input of the first operational amplifier (14) and to the non-inverting input of the second operational amplifier (15) by way of a first voltage divider (16, 17), and the negative operating voltage (-U_{V}) is supplied to the non-inverting input of the first operational amplifier (14) by way of a second voltage divider (19, 20), and the positive operating voltage (+U_{V}) is supplied to the inverting input of the second operational amplifier (15) by way of a third voltage divider (21, 22) which is dimensioned according to the second voltage divider.

2. Stimulator according to claim 1, characterised in that the percentage amount is approximately 90% of the operating voltage (±U_{V}).

3. Stimulator according to claim 1, characterised in that the input resistances (19, 21) of the second and third voltage dividers (19, 20; 21, 22) respectively connected to the negative and positive operating voltages (±U_{V}), are of equal size, and in that the input resistance (16) of the first voltage divider (16, 17) connected to the output voltage of the end stage (1) is in a relationship with each of the input resistances (19, 21) of the second and third voltage dividers (19, 20; 21, 22) which corresponds to the pre-selected percentage amount of operating voltage.

4. Stimulator according to claim 1, characterised in that the window discriminator (11) actuates the cut-out (4, 5, 10) by way of a bistable flip-flop (12).

5. Stimulator according to claim 1, characterised in that the tripping signal also activates an optical and/or acoustic alarm transmitter (13).

6. Stimulator according to one of claims 1 to 5, characterised in that, at the end stage (1), a signal output (3) can be disconnected from an electrode connection socket (6) by way of a first switch contact (4) of a relay (5), and a further switch contact (10) connects the electrode connection socket (6) to another electrode connection socket (7) in the manner of a short circuit when the relay (5) is activated in the sense of a disconnection of the inner electric circuit of the stimulator.

## Revendications

1. Stimulateur pour appliquer à un patient une thérapie à courant de stimulation, comportant un étage final (1) travaillant avec une tension de service (U_{V}) pouvant être prédéterminée et auquel est associé, côté sortie, un discriminateur à fenêtre (11), qui produit un signal d'interruption (S_{A}) pour un interrupteur (4,5,10) servant à interrompre un circuit interne du stimulateur, lorsque la composante continue de la tension à la sortie de l'étage final dépasse une valeur pouvant être prédéterminée, caractérisé par le fait qu'il est prévu un étage final (1), qui travaille en mode push-pull à une tension de service positive ou négative ((±U_{V}) pouvant être prédéterminée, et dans lequel le signal d'interruption (S_{A}) est produit lorsqu'une tension positive ou une tension négative (±U_{A}) présente à la sortie de l'étage final (1) dépasse une valeur en pourcentage pouvant être prédéterminée de la tension de service positive ou négative (±U_{V}), le discriminateur à fenêtre (11) comportant deux amplificateurs opérationnels (14, 15), dont les sorties sont reliées par l'intermédiaire de diodes (23, 24) pour produire le signal d'interruption (S_{A}), la tension de sortie (U_{A}) de l'amplificateur final (1) étant envoyée à l'entrée inverseuse du premier amplificateur opérationnel (14) et à l'entrée non inverseuse du second amplificateur opérationnel (15) par l'intermédiaire d'un premier diviseur de tension (16,17), et la tension de service négative (-U_{V}) étant envoyée à l'entrée non inverseuse du premier amplificateur opérationnel (14) par l'intermédiaire d'un second diviseur de tension (19,20), et la tension de service positive (+U_{V}) est envoyée à l'entrée inverseuse du second amplificateur opérationnel (15) par l'intermédiaire d'un troisième diviseur de tension (21,22), qui est dimensionné conformément au second diviseur de tension.

2. Stimulateur suivant la revendication 1, caractérisé par le fait que la valeur en pourcentage est égale à environ 90 % de la tension de service (±U_{V}).

3. Stimulateur suivant la revendication 1, caractérisé par le fait que les résistances d'entrée (19,21) des second et troisième diviseurs de tension (19,20; 21,22), qui sont reliés à la tension de service négative ou positive (±U_{V}), ont la même valeur et la résistance d'entrée (16) du premier diviseur de tension (16, 17), qui est reliée à la tension de sortie de l'étage final (1), est, par rapport à chacune des résistances d'entrée (19, 21) des second et troisième diviseurs de tension (19, 20; 21, 22), dans un rapport qui correspond à la valeur en pourcentage, pouvant être prédéterminée, de la tension de service.

4. Stimulateur suivant la revendication 1, caractérisé par le fait que le discriminateur à fenêtre (11) actionne l'interrupteur (4,5,10) par l'intermédiaire d'un étage à bascule bistable (12).

5. Stimulateur suivant la revendication 1, caractérisé par le fait que le signal d'interruption met en action également un générateur d'alarme visuel et/ou acoustique (13).

6. Stimulateur suivant l'une des revendications 1 à 5, caractérisé par le fait que dans l'étage final (1), une sortie de signaux (3) peut être débranchée d'une prise de raccordement d'électrode (6) par l'intermédiaire d'un premier contact de coupure (4) d'un relais (5), et qu'un autre contact de coupure (10) relie selon une liaison de court-circuit la prise de raccordement d'électrode (6) à une autre prise de raccordement d'électrode (7) lorsque le relais (5) est activé en vue d'un débranchement du circuit interne du stimulateur.
